(19) Europäisches Patentamt
European Patent Office
Office européen des brevets

(11) **EP 0 540 907 B1**

(12) **EUROPEAN PATENT SPECIFICATION**

(45) Date of publication and mention
of the grant of the patent:
**16.04.1997   Bulletin 1997/16**

(51) Int Cl.6: **G01N 27/416**

(21) Application number: **92117486.8**

(22) Date of filing: **29.07.1988**

(54) **Oxygen analyzer**

Sauerstoffanalysegerät

Analyseur d'oxygène

(84) Designated Contracting States:
**DE FR GB IT**

(30) Priority: **31.07.1987  JP  192415/87**
**31.07.1987  JP  192416/87**

(43) Date of publication of application:
**12.05.1993   Bulletin 1993/19**

(62) Application number of earlier application in
accordance with Art. 76 EPC: **88307035.1**

(73) Proprietor: **Osaka Sanso Kogyo Limited**
**Yodogawa-ku Osaka (JP)**

(72) Inventors:
• **Makihara, Akira**
**Funabashi-shi, Chiba-ken (JP)**
• **Matsumoto, Yoshiro**
**Funabashi-shi, Chiba-ken (JP)**

• **Kikuchi, Shigeru**
**Kamagaya-shi, Chiba-ken (JP)**

(74) Representative: **Ritter, Stephen David et al**
**Mathys & Squire**
**100 Grays Inn Road**
**London WC1X 8AL (GB)**

(56) References cited:
**FR-A- 2 111 421**          **GB-A- 1 272 619**
**GB-A- 2 140 563**          **US-A- 3 050 371**

• **PATENT ABSTRACTS OF JAPAN vol. 10, no. 206**
**(P-478)18 July 1986 & JP-A-61 047 548**

Remarks:
The file contains technical information submitted
after the application was filed and not included in this
specification

## Description

The present invention relates to a galvanic cell type oxygen analyzer. More particularly, the present invention is concerned with an oxygen analyzer wherein a specific metal is employed as a material for a water electrolyzing electrode of a standard gas generator for water electrolysis type calibration.

Generally, hydrogen is obtained by electrolysis of water or in the form of a gas produced as a by-product by iron works. When such a hydrogen gas is employed in the manufacture of semiconductor devices, a trace amount of oxygen contained in the hydrogen gas may impair the production of semiconductor devices. It is, therefore, necessary to measure the oxygen content in the hydrogen gas. It has heretofore been general practice to employ a galvanic cell type oxygen analyzer to measure the $O_2$ content in the hydrogen gas.

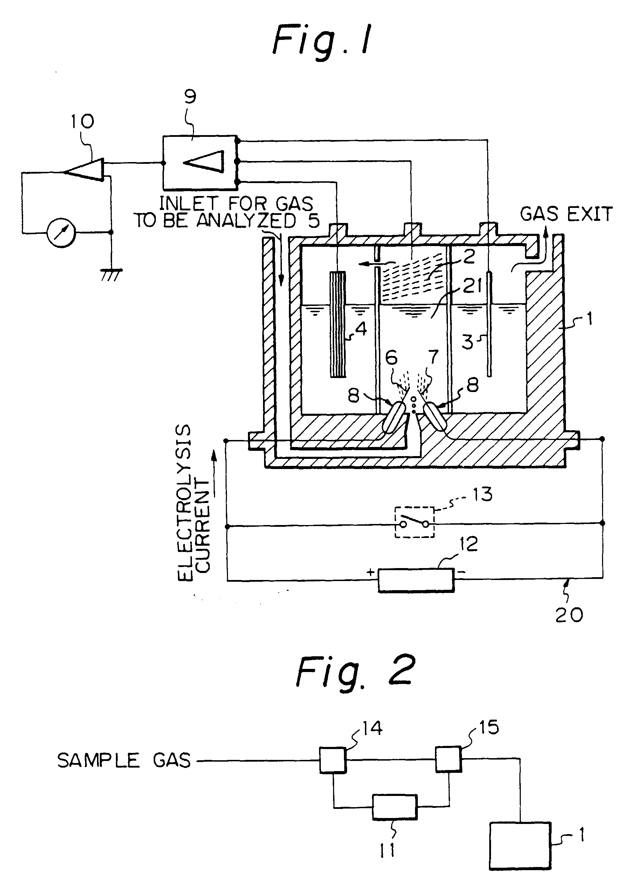

Fig. 1 schematically shows a known galvanic cell type oxygen analyzer. The following is a description of the principle of this galvanic cell type oxygen analyzer.

An analyzer cell 1 is a kind of galvanic cell which consists of a potassium hydroxide (20% KOH) electrolyte, and an inert silver (Ag) cathode 2, a protecting electrode 3 and an active cadmium (Cd) anode 4, all of which are immersed in the electrolyte. The silver electrode 2 is composed of about ten odd spirally arranged silver disks which are covered with a cylindrical tube. The cadmium electrode 4 has a relatively long and narrow cylindrical configuration. The protecting electrode 3 has a relatively long and narrow ribbon-shaped configuration. When a sample gas is supplied to this cell from a gas inlet 5, the gas passes through a wet tube in the center of the cell 1 to reach the surface of the silver electrode 2, and an amount of oxygen which is proportional to the oxygen partial pressure in the gas is adsorbed on the surface of the silver electrode 2. The adsorbed oxygen molecules dissolve in the electrolyte in the form of hydroxyl ions.

[Silver cathode]

$$O_2 + 2H_2O + 4e \rightarrow 4OH^- \qquad (1)$$

The hydroxyl ions move toward the cadmium anode 4 and oxidize cadmium to form cadmium hydroxide.

[Cadmium anode]

$$2Cd + 4OH^- \rightarrow 2Cd(OH)_2 + 4e \qquad (2)$$

When the hydroxyl ions lose their negative charges in this way, a current flows from the silver electrode 2 to an external circuit connected to the cadmium electrode 4. Accordingly, by measuring this current, it is possible to measure the oxygen concentration in the gas.

When the oxygen concentration is particularly high, the reactions of the formulae (1) and (2) proceed rapidly, so that the surface of the cadmium electrode 4 is oxidized quickly, resulting in lowering of the anode capacity. The protecting electrode 3 is provided in order to protect the cadmium electrode 4 from oxidation.

The protecting electrode 3 is supplied with the current flowing between the silver electrode 2 and the cadmium electrode 4 after it has been amplified.

Accordingly, most of the hydroxyl ions generated from the reaction of the formula (1) are attracted by the protecting electrode 3. Since the relatively small current flowing between the silver electrode 2 and the cadmium electrode 4 and the relatively large current flowing to the protecting electrode 3 are in proportional relation to each other, the current flowing to the protecting electrode 3 is utilized as an electric output from the cell 1.

In conventional galvanic cell type oxygen analyzers which have an anode protecting electrode, platinum, which is resistant to acids and alkalis, is employed as a material for the protecting electrode. However, it has been confirmed that the level of output noise increases somewhat when a trace amount of oxygen contained in a gas rich in hydrogen is measured with an oxygen analyzer employing platinum as a material for the anode protecting electrode. The smaller the oxygen content, the higher the noise level. Accordingly, the detectable minimum limit for the oxygen concentration in gases which are rich in hydrogen has heretofore been somewhat lower than that in the case of other inert gases (e. g., helium, argon, nitrogen, etc.) or combustible gases (methane, ethane, ethylene, etc.).

In general, calibration of a gas analyzer is effected using a zero gas filled in a vessel and a standard gas. In the case of oxygen standard gases, particularly those which contain a trace amount of oxygen, i.e., 1 ppm or less, the degree of precision lowers with the passage of time due to reaction with the surface of the vessel, degassing, etc. There is one type of trace oxygen analyzer generally known which has a purifier for removing the oxygen content from a gas which is to be measured to thereby obtain a zero gas and a standard gas generator for generating a standard oxygen gas by electrolysis of water. This type of oxygen analyzer suffers, however, from the disadvantage that it may be difficult or impossible to generate pure oxygen stably in a trace oxygen region depending upon the kind of sample gas employed.

When $H_2$, $N_2$ or Ar is employed as a zero gas in a calibration oxygen generator, there has heretofore been a risk that $O_2$ may not be normally generated from the anode of the oxygen analyzer. If platinum or the like is, by way of example, employed as a material for the anode or cathode of the oxygen generator, the amount of $O_2$ generated is reduced, although the mechanism responsible for this is not clear, so that it is impossible to obtain an $O_2$ gas for calibration having a desired concentration.

It is conventional practice from the viewpoint of thermal expansion to secure a platinum wire which serves as the anode or cathode of the oxygen analyzer through a soft glass. However, where the platinum wire is secured to the bottom of a detector cell, since an alkaline solution such as a potassium hydroxide, sodium hydroxide or potassium hydrogencarbonate solution is used as an electrolyte, the soft glass elutes with the passage of time, resulting in a gap at the interface between the glass and the platinum wire. The pure oxygen that is generated at this gap may form bubbles; in such a case, every time bubbles are liberated, the predetermined dilution ratio is destroyed, which makes it impossible to obtain a stable-state standard gas. There are cases where a hard glass which is considered to be only slightly soluble in an alkali is employed. However, in many cases, a crack develops at the interface between the glass and the platinum wire at the time of fusion welding due to the difference in the thermal expansion coefficients. Therefore, the production yield is low, and there is a fear of bubbles being generated, as described above.

On the other hand, there is a residual voltage between the anode and the cathode when no electrolysis is conducted, which promotes adhesion to the electrode surface of impurity metal ions in the water or the diluent gas, resulting in a film of impurities forming on the electrode surface. The resulting film of impurities obstructs normal electrolysis in a low oxygen concentration region.

The present inventors have found that the conventional anode protecting electrode formed by using platinum or the like readily causes dissociation and adsorption of hydrogen during analysis of a gas which is rich in hydrogen, resulting in an increase in the level of noise.

The present inventors conducted extensive studies and have found that employment of gold, silver or rhenium as one of the constituent materials of the anode protecting electrode of an oxygen analyzer enables a reduction in the noise that results from the dissociation and adsorption of hydrogen. The present invention and the invention of parent application EP 88307035.1 (EP 0 301 897B1) have been accomplished on the basis of this finding.

Thus EP 0 301 897B1 describes and claims:

- an oxygen analyzer comprising a galvanic cell with associated circuitry and being adapted to analyze oxygen in a hydrogen rich gas, said cell comprising first and second measuring electrodes acting respectively as an anode and a cathode, and at least one supplementary electrode arranged to intercept hydroxyl ions generated at the cathode so as to protect the anode from oxidation, characterised in that said supplementary electrode comprises an anode protecting electrode at least a surface portion of which comprises gold, silver or rhenium.

- a method of analyzing oxygen in a hydrogen-rich gas which comprises detecting the potential difference between an anode and a cathode in contact with an electrolyte exposed to said gas, wherein there is provided at least one supplementary electrode in contact with the electrolyte and arranged to intercept hydroxyl ions generated at the cathode so as to protect the anode from oxidation, characterized in that at least a surface portion of said supplementary electrode comprises gold, silver or rhenium.

The present invention provides an oxygen analyzer of the galvanic cell type, comprising calibration means which include an anode and a cathode arranged in use to contact an electrolyte exposed to a calibration gas and means for generating the calibration gas by electrolysis of water at an electrolyzing electrode, characterized in that at least a surface portion of said electrode comprises gold, silver or rhenium.

The invention further relates to a method of calibrating a galvanic cell for analyzing oxygen in hydrogen, nitrogen or argon gas which comprises detecting the potential difference between an anode and a cathode in contact with an electrolyte exposed to a calibration gas, wherein the calibration gas is generated by electrolysis of water at an electrolyzing electrode, characterized in that at least a surface portion of said electrode comprises gold, silver or rhenium.

Referring to the drawings:

Fig. 1 schematically shows a galvanic cell type oxygen analyzer;
Fig. 2 schematically shows a system for feeding a sample gas;
Fig. 3 is a graph showing the noise level in the case of an anode protecting electrode formed using platinum; and
Fig. 4 is a graph showing the noise level in the case of an anode protecting electrode formed using gold.

Preferable examples of the material for the anode protecting electrode of an oxygen analyzer or a water electrolyzing electrode of a standard gas generator for water electrolysis type calibration are gold, silver and rhenium. Among these, gold is the most preferable. Accordingly, it is preferable to make such electrodes of gold, silver or rhenium, or to use electrode materials which are formed by coating the reaction surface or the whole surface of a metal (e.g., copper or nickel), ceramic or resin material with gold, silver or rhenium by means, for example, of sputtering. These metals may, of course, be coated by other methods.

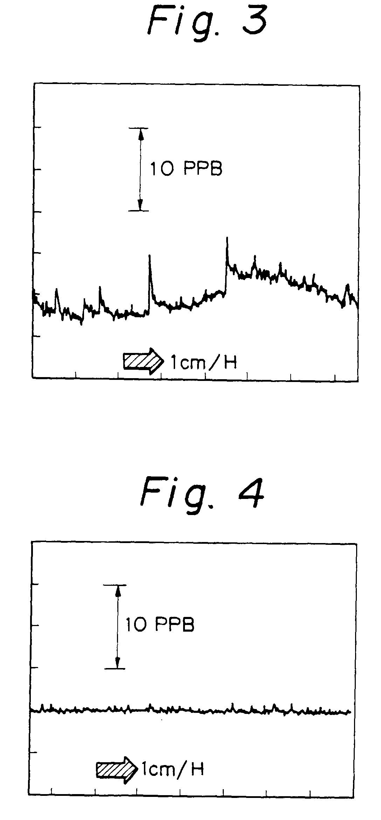

An anode protecting electrode was experimentally formed using gold, and the noise level was measured in the range of 100 PPB. The noise level in the case of the gold anode-protecting electrode was ±1 PPB or less, whereas the noise level in the case of a platinum anode-protecting electrode was about ±5 PPB. The results of the measurement are shown in Figs. 3 and 4. Fig. 3 is a graph showing the noise level in the case of the platinum anode-protecting electrode, while Fig. 4 is a graph showing the noise level in the case of the gold anode-protecting electrode. As will be understood from these graphs, employment of gold as a material for the anode protecting electrode enables the noise level to be lowered to about one fifth of that in the case of a platinum anode-protecting electrode.

As described above, the present invention enables the noise generated in the measurement of oxygen contained in a trace amount in a gas rich in hydrogen to be lowered to the same level as in the case of other inert gases or combustible gases. Thus, it is possible to measure precisely the concentration of oxygen contained in a trace amount in a gas which is rich in hydrogen.

It should be noted that the arrangement shown in Fig. 1 is also provided with a calibration circuit 20 for generating an oxygen standard gas by electrolysis of water. The reference numeral 6 denotes an anode, 7 a cathode, 8 epoxy resin sleeves, and 9, 10 amplifiers.

Fig. 2 shows a system for feeding a sample gas for measurement. At the time of analysis of $O_2$, the sample gas is not passed through an $O_2$ purifier 11 but is supplied to the gas inlet 5 of the cell 1 through valves 14 and 15. When calibration of the oxygen analyzer is to be conducted, the sample gas is passed through the $O_2$ purifier 11 where oxygen is completely removed, and then introduced into the cell 1 from the inlet 5 as an "oxygen zero gas". The reference numeral 12 denotes a power supply, and 13 a switch. When a current is passed through the circuit 20, $O_2$ and $H_2$ are generated from the anode 6 and the cathode 7, respectively. The pure $O_2$ thus generated is diluted with the oxygen zero gas introduced from the gas inlet 5 to thereby generate a standard gas having a predetermined oxygen concentration.

Referring to Fig. 1, the oxygen analyzer according to the present invention comprises a pair of water electrolyzing electrodes, epoxy resin sleeves, and a switch for shorting these two electrodes. The present invention will be described hereinunder in detail with reference to Fig. 1 which shows the structure of the oxygen analyzer. The reference numerals 6 and 7 denote a pair of water electrolyzing electrodes. That portion of each of the electrodes 6 and 7 which is in contact with an electrolyte is formed using gold, silver or rhenium, preferably gold, which is the noblest metal. The electrodes 6 and 7 may have any kind of configuration, e.g., a linear, rod- or band-shaped configuration. The distal end of each electrode may be coiled. It is also possible to form these electrodes 6 and 7 by coating an electrically conductive material, e.g., copper or nickel, with gold, silver or rhenium by means of, for example, sputtering, vacuum evaporation or electroplating. Osmium causes less dissociation and adsorption of hydrogen but has inferior workability and is therefore unsuitable for use as an electrode material. If an electrolytic current is passed in the illustrated direction, pure oxygen and pure hydrogen are generated by the electrodes 6 and 7, respectively. The pure oxygen generated by the electrode 6 is diluted with the oxygen zero gas introduced from the inlet 5 to thereby obtain an oxygen standard gas. When hydrogen is, by way of example, employed as an oxygen zero gas, an electrode formed from gold, silver or rhenium is unlikely to cause dissociation and adsorption of hydrogen at its surface and therefore exhibits excellent water electrolysis characteristics in a trace oxygen region. In addition, the period of time from the instant the application of a constant current is started until the generation of pure oxygen begins is shorter than that in the case of an electrode formed using platinum or other platinum-based noble alloys, and therefore the electrode of gold, silver or rhenium exhibits excellent stability.

The reference numeral 1 denotes a vessel for electrolysis of water which may also serve as a detector cell and/or humidifier. The vessel 1 is formed from a material which is selected from the group consisting of epoxy resins, vinyl chloride and hard glass, preferably an epoxy resin which adheres most strongly to the epoxy resin sleeves 8 from the viewpoint of such aspects as resistance to alkalis, gas barrier properties and electrical insulating properties.

The reference numeral 21 denotes an alkaline solution such as a potassium hydroxide, sodium hydroxide or potassium hydrogencarbonate solution.

The numeral 8 denotes epoxy resin sleeves. An electrode material is received through each cylindrical epoxy resin sleeve 8 and bonded thereto by means of an epoxy resin adhesive. Further, the epoxy resin sleeves 8 are secured to the vessel 1 for electrolysis of water by means of an epoxy resin adhesive. Many epoxy resins have their setting temperatures adjusted to ordinary temperature by being mixed with a hardener. Therefore, employment of an epoxy resin as a material for the vessel 1 for water electrolysis and the sleeves 8 requires no heating or cooling steps in the process for securing the electrodes. Accordingly, no cracks are generated at the interface between the electrode material and the epoxy resin which would otherwise be developed due to the difference in thermal expansion coefficients. In other words, the pure oxygen which is generated by electrolysis of water is effectively diffused into the electrolyte without producing bubbles.

The reference numeral 13 denotes a switch. The switch 13 is provided on the circuit between the anode and the cathode which are defined by the water electrolyzing electrodes. When electrolysis is being conducted, the switch 13 is opened, whereas when no electrolysis is taking place, the switch 13 is closed. Thus, there is no residual voltage between the two electrodes when no electrolysis is being conducted. Accordingly, it is possible to prevent adhesion of impurity metal ions to the electrode surface, and it becomes easy to conduct water electrolysis in a trace oxygen region.

The reference numeral 20 denotes a constant-current circuit.

The present invention enables pure oxygen to be readily and stably generated in a trace oxygen region even in the case of a hydrogen-rich diluent gas. In addition, it is possible to prevent production of bubbles of pure oxygen at the interface between the electrode material and the epoxy resin sleeve, and therefore there is no risk of the predetermined dilution ratio being destroyed. Accordingly, it is possible to obtain a standard gas having a stable oxygen concentration. It is also possible to prevent the formation of a film on the electrode surface by impurity metal ions, and it is therefore possible to conduct stable electrolysis of a hydrogen-rich gas which contains a trace amount of oxygen.

Fig. 2 shows a system for feeding a sample gas for measurement. At the time of analysis of $O_2$, the sample gas is not passed through the $O_2$ purifier 11 but is supplied to the gas inlet 5 of the cell 1 through valves 14 and 15. When calibration of the oxygen analyzer is to be conducted, the sample gas is passed through the $O_2$ purifier 11 where $O_2$ is completely removed therefrom, and is then introduced into the cell 1 from the inlet 5 as an oxygen zero gas.

## Claims

1. An oxygen analyzer of the galvanic cell type, comprising calibration means which include an anode and a cathode arranged in use to contact an electrolyte exposed to a calibration gas and means for generating the calibration gas by electrolysis of water at an electrolyzing electrode, characterized in that at least a surface portion of said electrode comprises gold, silver or rhenium.

2. An oxygen analyzer according to Claim 1, wherein said water electrolyzing electrode is formed using a material prepared by coating an electrically conductive material, for example, nickel or copper, with gold, silver or rhenium by means, for example, of sputtering, vacuum evaporation or electroplating.

3. An oxygen analyzer according to Claim 1 or Claim 2, wherein said water electrolyzing electrode is secured to an electrolytic cell by means of bonding through an epoxy resin sleeve.

4. An oxygen analyzer according to any of Claims 1 to 3, wherein a switch is provided on the circuit between an anode and a cathode, each of which is defined by said water electrolyzing electrode.

5. A method of calibrating a galvanic cell for analyzing oxygen in hydrogen, nitrogen or argon gas which comprises detecting the potential difference between an anode and a cathode in contact with an electrolyte exposed to a calibration gas, wherein the calibration gas is generated by electrolysis of water at an electrolyzing electrode, characterized in that at least a surface portion of said electode comprises gold, silver or rhenium.

## Patentansprüche

1. Galvanikzellen-Sauerstoffanalysator mit einer Kalibriervorrichtung, welche eine Anode und eine Kathode enthält, die im Gebrauch so angeordnet sind, daß ein Elektrolyt berührt wird, der einem Kalibriergas ausgesetzt ist, sowie eine Vorrichtung zur Erzeugung des Kalibriergases durch Elektroyse von Wasser an einer Elektrolyseelektrode, dadurch **gekennzeichnet**, daß zumindest ein Oberflächenabschnitt der Elektrode Gold, Silber oder Rhenium aufweist.

2. Sauerstoffanalysator nach Anspruch 1, bei welchem die Wasserelektrolyseelektrode unter Verwendung eines Materials hergestellt ist, welches durch Beschichtung eines elektrisch leitfähigen Materials, beispielsweise Nickel oder Kupfer, mit Gold, Silber oder Rhenium mit Hilfe beispielsweise von Sputtern, Vakuumverdampfung oder Elektroplattierung hergestellt wird.

3. Sauerstoffanalysator nach Anspruch 1 oder 2, bei welchem die Wasserelektrolyseelektrode an einer Elektrolysezelle mit Hilfe einer Verbindung über eine Epoxyharzmuffe befestigt ist.

4. Sauerstoffanalysator nach einem der Ansprüche 1 bis 3, bei welchem ein Schalter in der Schaltung zwischen einer Anode und einer Kathode vorgesehen ist, die jeweils durch die Wasserelektrolyseelektrode festgelegt werden.

5. Verfahren zum Kalibrieren einer Galvanikzelle zur Analyse von Sauerstoff in Wasserstoff-, Stickstoff- oder Argongas, mit Feststellung der Potentialdifferenz zwischen einer Anode und einer Kathode in Berührung mit einem Elektrolyten, der einem Kalibriergas ausgesetzt ist, wobei das Kalibriergas durch Elektrolyse von Wasser an einer Elektrolyseelektrode erzeugt wird, dadurch **gekennzeichnet**, daß zumindest ein Oberflächenabschnitt der Elektrode Gold, Silber oder Rhenium enthält.

## Revendications

1. Analyseur d'oxygène du type à cellule galvanique, comprenant un moyen de calibrage qui comprend une anode et une cathode agencées en utilisation pour contacter un électrolyte exposé à un gaz de calibrage et un moyen pour produire le gaz de calibrage par électrolyse d'eau à une électrode d'électrolyse, caractérisé en ce qu'au moins une portion de surface de ladite électrode comprend de l'or, de l'argent ou du rhénium.

2. Analyseur d'oxygène selon la revendication 1, dans lequel l'électrode d'électrolyse (2) est formée en utilisant un matériau préparé en revêtant un matériau électriquement conducteur, par exemple, du nickel ou du cuivre, avec de l'or, de l'argent ou du rhénium au moyen, par exemple, de pulvérisation, d'évaporation sous vide ou d'électro-déposition.

3. Analyseur d'oxygène selon la revendication 1 ou la revendication 2 dans lequel l'électrode d'électrolyse d'eau précitée est fixée à une cellule électrolytique au moyen d'une liaison par un manchon à résine époxy.

4. Analyseur d'oxygène selon l'une quelconque des revendications 1 à 3, dans lequel un commutateur est prévu sur le circuit entre une anode et une cathode, dont chacune est définie par l'électrode d'électrolyse d'eau précitée.

5. Procédé de calibrage d'une cellule galvanique pour analyser de l'oxygène dans de l'hydrogène, de l'azote ou du gaz argon qui comprend détecter la différence de potentiel entre une anode et une cathode en contact avec une électrode exposée à un gaz de calibrage, où le gaz de calibrage est produit par électrolyse d'eau à une électrode d'électrolyse, caractérisé en ce qu'au moins une portion de surface de ladite électrode comprend de l'or, de l'argent ou du rhénium.

# Fig. 1

INLET FOR GAS TO BE ANALYZED 5

GAS EXIT

ELECTROLYSIS CURRENT

# Fig. 2

SAMPLE GAS

# Fig. 3

10 PPB

1cm/H

# Fig. 4

10 PPB

1cm/H